# EUROPEAN PATENT APPLICATION

(11) **EP 4 751 666 A1**
(43) Date of publication of application: **03.06.2026**
(21) Application number: 24216112.3
(22) Date of filing: 28.11.2024
(51) Int. Cl.: A61B 17/66, A61B 17/84, A61B 17/88

(54) **BONE DEFORMITY REPAIRING SYSTEM**

(71) Applicant: BRM Extremities S.r.l., 20145 Milano (MI) (IT)
(72) Inventor: Rocha de Souza, André Luiz, 49038443 Aracaju (BR)
(74) Representative: Dragotti & Associati S.P.A.

(57) **Abstract**

A bone deformity repairing system comprises a dorsal gap reducer (1) comprising a first anchoring block (10) that is configured to rotatably anchor the dorsal gap reducer (1) to a first bone, a gap reducing block (20) that is slidably connected to the first anchoring block (10) and a second anchoring block (30) that is rotatable with respect to and selectively lockable to the gap reducing block (20) and it is configured to be anchored to a second bone.

## Description

The present invention relates to the field of medical devices and more specifically to medical devices configured to be used in orthopedic surgery. The present invention concerns a bone deformity repairing system designed for the repair of bone deformities in the foot, and more particularly, designed for a Lapidus procedure.

The field of medical devices configured to be used in orthopedic surgery encompasses a range of systems designed to correct skeletal deformities, particularly those affecting the bones and joints of the foot. A common procedure in this field is the surgical correction of bones deformities, which frequently involves realignment of bone structures to achieve functional and anatomical improvements. Document US 20240058017A1 introduces an advanced approach to such surgical interventions, describing a system that incorporate one or more guides to facilitate precise positioning of instruments in relation to bones or joints during operations like a Lapidus procedure. The disclosed system exemplifies attempts in the prior art to enhance surgical precision through sophisticated instrument guides. It features a cutting guide that includes an adjustable guide sleeve and block along a drive shaft, enabling surgeons to fine-tune the position of the guide sleeve with respect to the patient's anatomy. Additionally, it discusses a targeting guide composed of a carrier coupled with housing and a pivotably connected guide sleeve, which can be crucial for achieving accurate placement of fixation elements across joints.

Despite these advancements, there remain inherent challenges associated with existing technologies. One such challenge is reducing space or 'gaps' between bone segments -particularly relevant when addressing dorsal dislocations or malalignments commonly encountered during foot surgeries. Present methods may not adequately address intraoperative reduction needs while simultaneously accommodating various anatomical variations without compromising stability or healing outcomes.

Another difficulty prevalent in current techniques is ensuring proper alignment during corrective procedures while effectively positioning fixing elements like screws or plates. The ability for these elements to maintain their position throughout the healing process is paramount for successful recovery and restoration of joint function. Moreover, existing solutions may lack integrative approaches that streamline multiple stages of bone correction into one cohesive system. Such integration could potentially allow for increased efficiency during surgery as well as improved postoperative recovery by providing a more comprehensive solution tailored toward different aspects of bone deformity repair within one platform.

The application of these principles within orthopedic interventions suggests an ongoing pursuit for tools that offer surgeons greater control over operative outcomes and patient mobility post-surgery. Systems that integrate gap reduction mechanisms with precise positioning guides for fixing elements are viewed as beneficial next steps toward enhancing existing methodologies used in bone deformity repair surgeries. Thus far, prior art has laid foundational work by introducing concepts such as adjustable cutting guides and pivotable sleeves in targeting guides which set benchmarks in terms precision tooling during orthopedic procedures. However, it remains evident that further refinements are necessary - particularly those that consider simultaneous gap reduction alongside alignment correction - to improve upon currently available technologies. Traditional methods often result in imprecise resection of articular cartilage, increased surgical time, and higher radiation exposure.

It is therefore an object of the present invention to provide a system, configured to be used in orthopedic surgery procedures for repairing bone deformities, that offers improved precision, adaptability, and effectiveness in correcting various types of bone misalignments and deformities.

Another object of the present invention is to provide a system that provides precise alignment and stabilization during a surgical procedure of correction of bone deformities, thereby facilitating effective and accurate treatment.

More in detail, the present invention addresses the problem of improving the precision and efficiency of the Lapidus procedure, which is used to treat hallux valgus deformity by fusing the tarsometatarsal (TMT) joint. The invention solves these issues by providing a system that enhances the precision of cartilage resection, reduces surgical time, and ensures better positioning of the first ray in the sagittal and coronal planes, while also minimizing radiation exposure.

In view of these objects, the Applicant has decided to create, according to the invention, a bone deformity repairing system as defined in claim 1.

The dependent claims outline preferred and/or particularly advantageous aspects of the invention.

Further features and advantages of the present invention will become more evident from the following description, made by way of example with reference to the attached figures in which:
- Figure 1 is a perspective view of a dorsal gap reducer according to the present invention;
- Figure 2 is a see-through view of the dorsal gap reducer of Figure 1;

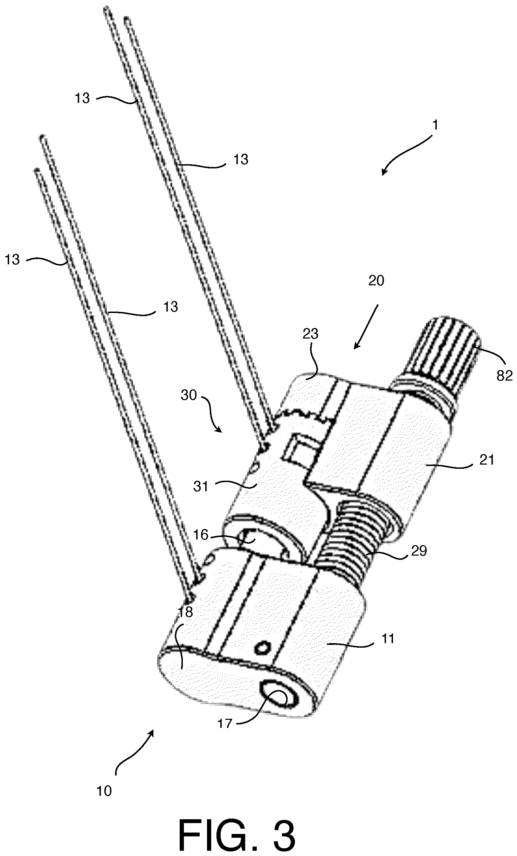

- Figure 3 is the same perspective view of Fig. 1 with the dorsal gap reducer in an operative position;

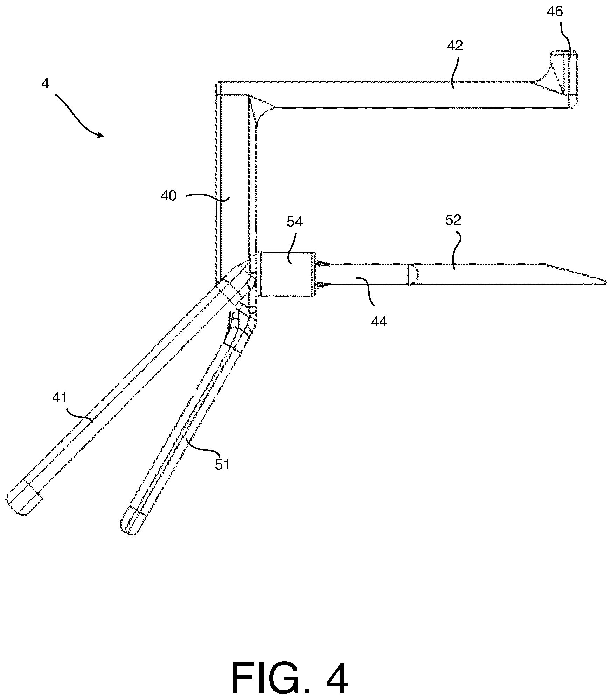

- Figure 4 is a lateral view of a positioning guide according to the present invention;

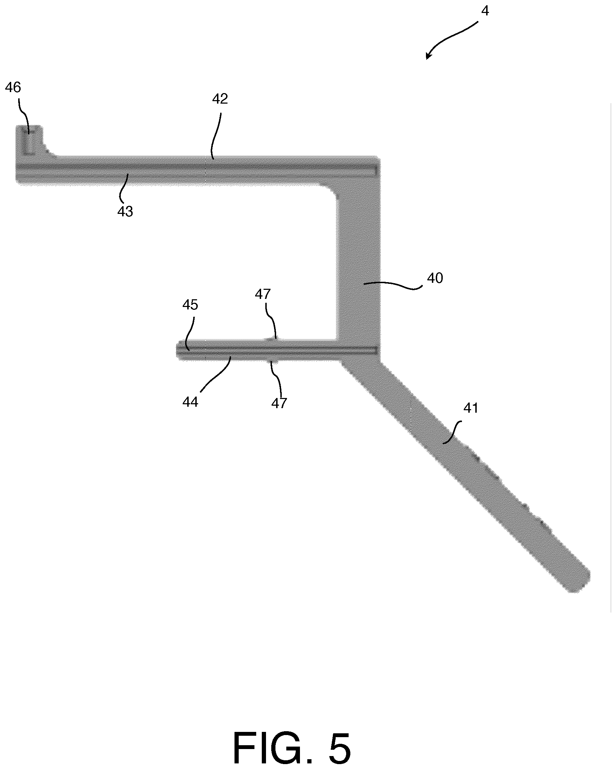

- Figure 5 is a sectional view of the guide of Figure 4;

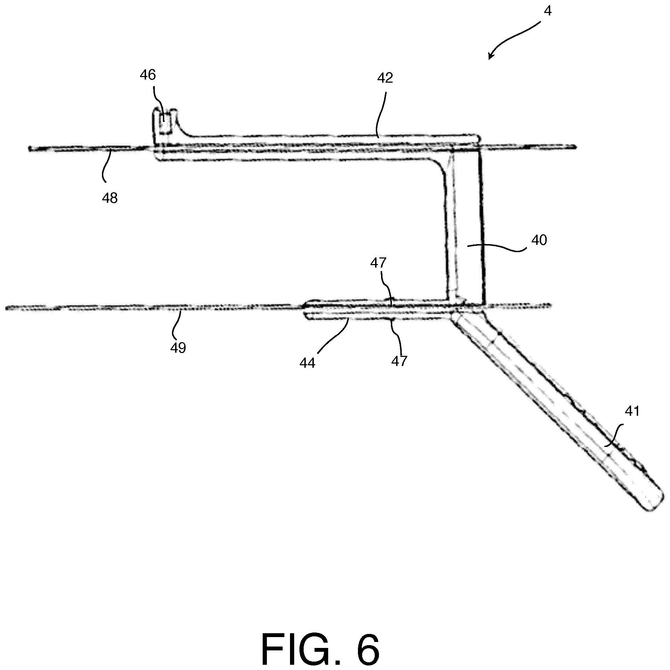

- Figure 6 is the same sectional view of Figure 5 with the guide in in an operative position;

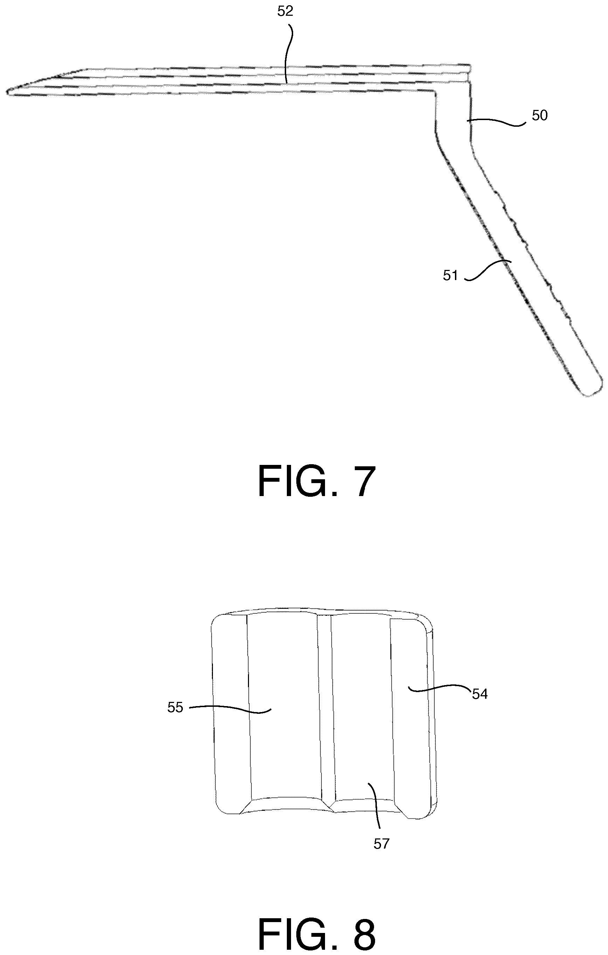

- Figure 7 is a sectional view of a secondary body of the guide of Figure 4;
- Figure 8 is a schematical view of a retaining element of the guide of Figure 4;

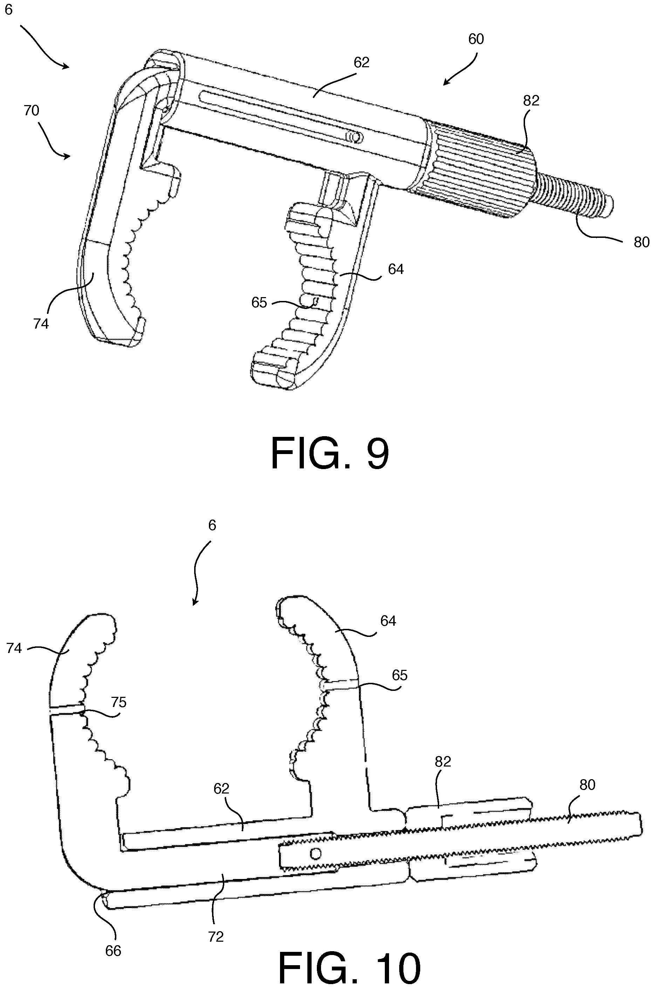

- Figure 9 is a perspective view of an alignment corrector according to the present invention; and
- Figure 10 is a sectional view of the alignment corrector of Figure 9.

It should be understood that elements and features of one embodiment can be conveniently incorporated into other embodiments without further clarification.

Reference will now be made in detail to the various embodiments of the invention, of which one or more examples are illustrated in the attached figures. Each example is provided purely by way of illustration of the invention and is not intended as a limitation thereof. For example, the technical features illustrated or described as forming part of an embodiment may be adopted on, or in association with, other embodiments to produce a further embodiment. It is understood that the present invention will include these modifications and variations.

A bone deformity repairing system according to a first embodiment of the present invention may comprise a dorsal gap reducer, generally indicated with 1.

The term "dorsal gap reducer" refers to a device used in the system of the present invention for repairing bone deformities. In a broad sense, the device is configured to reduce a gap between bones and to restore alignment between bones, specifically between two bones connected to each other through a join. In a narrow sense, the dorsal gap reducer comprises three main components: a first anchoring block that is configured to be rotatably anchored to a first bone, a gap reducing block that is slidably connected to the first anchoring block and a second anchoring block that is selectively rotatably connected to the gap reducing block and it is configured to be anchored to a second bone. This configuration allows for precise adjustment and alignment of the bones during a repairing process.

In other words, the dorsal gap reducer is configured to addresses the common issue of excessive space between bones and of misalignment between bones in deformity conditions, facilitating proper healing.

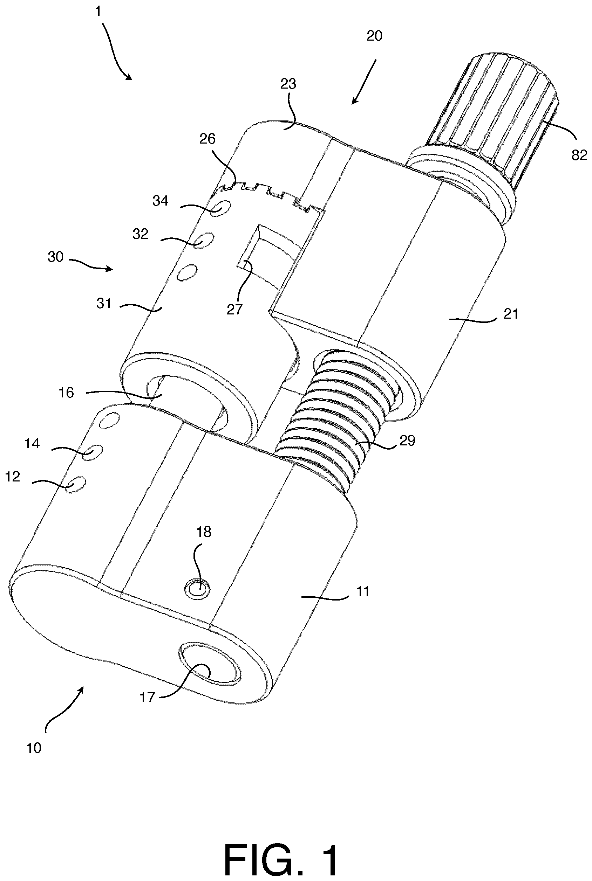

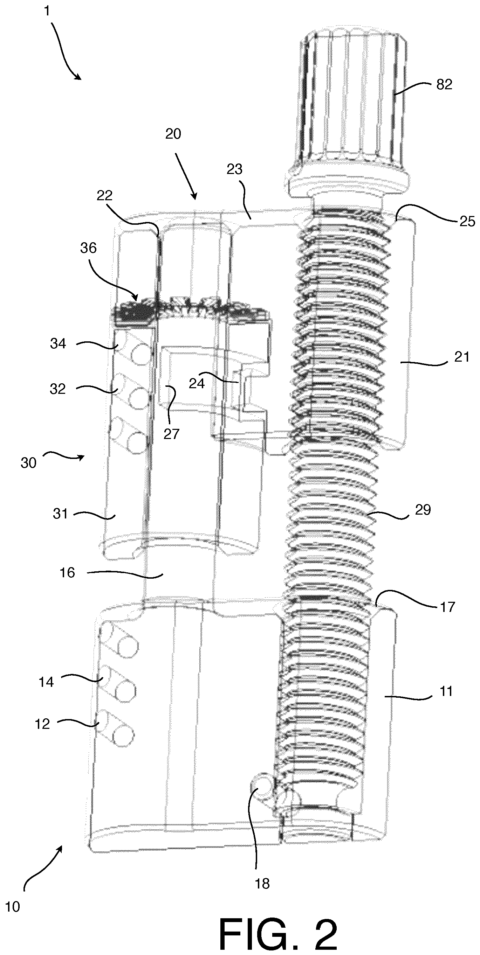

With reference to Figs. 1 and 2, a dorsal gap reducer 1 according to the present invention may comprise a first anchoring block 10 configured to be anchored to a first bone. The first anchoring block 10 may comprise a main body 11 and an anchoring group configured to rotatably anchoring the dorsal gap reducer 1 to a first bone. According to embodiment of the present invention, the first bone may be a tarsal bone of a foot. The term "tarsal bone" refers to any of the seven bones in the human foot that form the ankle and upper part of the foot.

According to the embodiment shown in the figures, the anchoring group comprises at least two holes 12, 14 provided at a first extremity portion of the main body 11 of the first anchoring block 10 and at least two anchoring elements configurated to selectively anchor the main body 11 to the first bone. The holes 12, 14 are configured to accommodate the anchoring elements, for example surgical wires or pins 13. If only a wire 13 is accommodated in a hole 12 and inserted into the first bone, the dorsal gap reducer 1 may be rotated with respect to the bone, as it will appear clearer hereinafter.

This configuration may allow for precise positioning and adjustment of the dorsal gap reducer 1 in relation to a tarsal bone, which is often involved in foot deformities. The rotatable anchoring may provide flexibility in accommodating various bone shapes and sizes, as well as allowing for fine-tuning of the reduction process.

Of course, different type of anchoring group, with respect to the embodiment previously described and shown in the figures, could be adopted as far as they are configured to selectively rotatably anchoring the dorsal gap reducer 1 to a first bone and provide the above-mentioned advantages.

The first anchoring block 10 may comprise a connecting bar 16 longitudinally protruding from the main body 11 with a predetermined length.

The first anchoring block 10 may comprise a seat 17 configured to receive an actuating element, as it will be clearer herein after.

The dorsal gap reducer 1 may comprise a gap reducing block 20 configured to be slidably connected to the first anchoring block 10, so that, in use, it is possible to reduce, or increase, the distance between the first 10 anchoring block and the gap reducing block 20.

According to the embodiment shown in the Figures 1 and 2, the gap reducing block 20 may comprise a main body 21 having a hollow peripheral portion 23 provided with a seat 22. In order to slidably connect the first anchoring block 10 to the gap reducing block 20, the connecting bar 16 of the first anchoring block 10 is inserted in the seat 22 of the hollow peripheral portion 23.

This slidable connection may enable smooth and controlled movement between the first anchoring block 10 and the gap reducing block 20, configured to allow a gradual and precise adjustment of the gap between bones, as it will be clearer herein after.

The dorsal gap reducer 1 may comprise a second anchoring block 30 configured to anchoring the gap reducing block 20 to a second bone.

The second anchoring block 30 is rotatably connected to and selectively lockable to the gap reducing block 20.

According to the embodiment shown in the figures, the second anchoring block 30 comprises a cylindrical component 31 slidably and rotatably disposed on the longitudinal connecting bar 16 inserted in the gap reducing block 20.

The cylindrical component 31 of the second anchoring block 30 may comprise at least one hole, preferably, two holes 32, 34, configured to accommodate anchors, for example surgical wires or pins 35, anchoring the second anchoring block 30 to a second bone.

The second anchoring block 30 is also selectively lockable to the main body 21 of the gap reducing block 20.

According to the embodiment shown, a lateral side of the cylindrical component 31 comprises an indentation 36 circumferentially distributed on said side. The external surface of the hollow peripheral portion 23 of the gap reducing block 20 is provided with an indentation 26 circumferentially distributed and configured to match the indentation 36 of the cylindrical component 31.

Therefore, when the second anchoring block 30 is abutted against the hollow peripheral portion 23 and the two indentations 26, 36 match to each other, the second anchoring group 30 is integral with the gap reducing block 20, while when the second anchoring block 30 is separated from the hollow peripheral portion 23, it is free to rotate with respect to both the first 10 anchoring group and the gap reducing block 20.

Moreover, according to this configuration, when the second anchoring block 30 is anchored to the second bone through the surgical pins 13, and the cylindrical component 31 is not abutted against the peripheral portion 23 of the gap reducing block 20, the cylindrical component 31 may rotate on the connecting bar 16 and with respect to the main body 21, and therefore radially modify the axial displacement of the second bone with respect to the first bone.

According to an embodiment of the present invention, the cylindrical component 31 of the gap reducing block 20 comprises a radial slot 27 and the main body 21 of the gap reducing block 20 comprises a prominence 24 disposed space apart from the hollow peripheral portion 23 and inside the slot 27. This configuration allows the cylindrical body 31 to slide on the longitudinal connecting bar 16 and the second anchoring block 30 to be unlocked from the gap reducing block 20, and, at the same time, it prevents the cylindrical body 31 to slide too far towards the first anchoring block 10 and it prevents a 360° rotation of the second anchoring block 30.

According to embodiments of the present invention, the second bone may be a metatarsal bone of a foot. The term "metatarsal bone" refers to any of the five long bones in the foot that connect the tarsal bones to the phalanges (toe bones).

This anchoring to both the tarsal and metatarsal bones enables the dorsal gap reducer of the present invention to provide comprehensive correction across the midfoot region. These rotatable connections of the anchoring blocks 10, 30 to the tarsal and metatarsal bones respectively may provide a high degree of flexibility in positioning and adjusting the bone deformity repairing system. Such flexibility may be beneficial in addressing complex deformities that require multi-directional correction. According to other embodiments, the connecting configuration of the first anchoring block 10 and the gap reducing block 20 may be reversed. For example, the gap reducing block 20 may comprise a connecting bar 16 longitudinally protruding from the main body 21 and the first anchoring block 10 may comprise a hollow portion with a seat provided in the main body 11, in order to slidably connect the first anchoring block 10 to the gap reducing block 20.

The dorsal gap reducer 1 may further comprise an actuating mechanism configurated to slide the gap reducing block 20 with respect to the first anchoring block 10, so as to reduce or increase a reciprocal distance therebetween.

According to the embodiment shown in the figures, the actuating mechanism may comprise a threaded rod 29 accommodated in a threaded conduit 25 provided on the gap reducing block 20 and inserted in the seat 17 of the first anchoring block 10. In use, rotating the threaded rod 29 in a first direction brings the gap reducing block 20 closer to the first anchoring block 10, and rotating the threaded rod 29 in a second opposite direction brings the gap reducing block 20 away from the first anchoring block 10.

The actuating mechanism may be configured to withstand the forces involved in bone reduction while maintaining ease of use for the surgeon.

The first anchoring block 10 may also comprise a though-hole 18 facing on the seat 17 and configured to accommodate a pin. The pin may be configured to abut against the threaded rod 29 in order to avoid the threaded rod 29 to be extracted from the seat 17, and thus to avoid disconnection between the first anchoring block 10 and the gap reducing block 20. The pin may also be configured to stop further rotations of the threaded rod 29 and thus fix the relative position of the two anchoring blocks 10, 20. According to further embodiments, the bone deformity repairing system of the present invention may also comprise a positioning guide, generally indicated with 4.

The term "positioning guide" refers to a tool or device used to assist in the accurate placement of one or more fixing elements during the bone repair process. In a broad sense, it ensures that the fixing elements, such as screws or pins, are positioned correctly to achieve optimal alignment and stabilization of the bones. In a narrow sense, the positioning guide is specifically designed to work in conjunction with the dorsal gap reducer, providing a system for repairing bone deformities ensuring that fixation elements are placed in optimal locations for long-term stability and proper healing.

The positioning guide 4 may comprise a main body 40 comprising at least two longitudinal arms 42, 44, orthogonally extending from the main body 40 and vertically superimposed to each other. Both the longitudinal arms 42, 44 may be hollow and may comprise a channel 43, 45 respectively.

The upper arm 42 has a length greater than the second lower arms 44 and it is configured to house an alignment element 48, for example a Kirschner wire.

The upper arm 42 further comprises at one end a blind hole 46 configured to house a pin. The pin may be configured to be used to visualizing, together with the wires, a correct alignment of the positioning guide (before inserting a fixing element). Preferably, the pin is made of metal to allow an X-ray visualization.

The lower arm 44 is configured to house a fixing element, for example a surgical pin or wire 49. The lower arm 44 may further comprise a retention element, for example a couple of projections 47, disposed on the external surface of the arm 44. According to the embodiment show in the figures, the main body 40 may comprise a handle 41, extending from the main body 40 under the lower arm 44 and inclined with respect to the main body 40.

The position guide 4 may further comprise a secondary body 50 comprising third hollow arm 52, orthogonally extending from the secondary body 50, and configured to house a second fixing element, for example a surgical pin or wire.

The secondary body 50 is configured to be coupled to the main body 40 by a retaining element, for example a ring 54. The retaining element 54 comprises two seats 55, 57 on its internal surface where the lower arm 44 and the third hollow arm 52 can be housed. According to the embodiment shown in the drawings, the retaining element 54 may made by two semi-shells coupled together once the lower arm 44 and the third hollow arm 52 have been positioned in the seats 55, 57. The retaining element 54 is retained to the lower arm 44, and thus to the main body 40, by the retention element 47, 47'.

The secondary body 50 can coupled both on the right side or the left side of the main body 50 so as to be configured to be used on either a right or left foot.

According to the embodiment show in the figures, also the secondary body 50 may comprise a handle 51, extending from the secondary body 50 under the lower arm 44 and inclined with respect to the secondary body 50.

According to further embodiments, the bone deformity repairing system according to other embodiments of the present invention may also comprise an alignment corrector, generally indicated with 6.

In these embodiments the system may comprise the alignment corrector 6 alone, or the dorsal gap reducer 1 and the alignment corrector 6, or the dorsal gap reducer 1, the alignment guide 4 and the alignment corrector 6.

The term "alignment corrector" refers to a device or mechanism used to correct the alignment of bones during a repairing process. In a broad sense, it ensures that the bones are properly aligned to promote healing and restore normal function. In a narrow sense, the alignment corrector may works in conjunction with the dorsal gap reducer and positioning guide to provide a precise and controlled method for correcting bone deformities. It may include features such as adjustable components or markers to guide the alignment process.

The alignment corrector may be an additional feature of the system. This component may be designed to address any residual misalignment after the initial reduction and fixation. The alignment corrector may allow for fine-tuning of bone position, ensuring that the final alignment is as close to anatomical normal as possible.

The alignment corrector 6 may comprise a first jawing block 60 and a second jawing block 70 slidably connected to the first jawing block 60.

According to the embodiment shown in the figures, the first jawing block 60 comprises a longitudinal hollow main body 62 comprising a longitudinal seat 66, and a first arm 64 vertically extending from the longitudinal hollow main body 62 and having a rounded shape configured to accompany the anatomy of a foot.

The second jawing block 70 comprises a longitudinal un-hollow body 72 and a second arm 74 vertically extending from the longitudinal un-hollow body 72 and having a rounded shape configured to accompany the anatomy of a foot. The shape of the second arm 74 being specular to the shape of the first arm 72. The un-hollow body 72 is housed in the longitudinal seat 66 of the longitudinal hollow main body 62 of the first jawing block 60.

According to embodiments, a through hole 65, 75 is provided in the central portion of both the first arm 64 and the second arm 74. The holes 65, 75 are configured to accommodate a K-wire, passing though both the holes 65, 75, which is used to fix the varus corrector to the second bone, i.e. a metatarsus one, a thus increasing the stability of the alignment corrector 6.

The alignment corrector 6 may further comprise an actuating mechanism configurated to slide the second jawing block 70 along the longitudinal hollow main body 62, so as to reduce or increase the reciprocal distance between the jawing blocks 60, 70.

According to the embodiment shown in the figures, the actuating mechanism comprises a threaded rod 80 inserted in the longitudinal hollow main body 62 of the first jawing block 60 and connected to the un-hollow body 72 of the second jawing block 70. A knob 82 may be fitted at one end of the threaded rod 80 in order to facilitate the rotation thereof.

In use, rotating the threaded rod 80 in a first direction brings the second arm 74 closer to the first arm 64, and rotating the threaded rod 80 in a second opposite direction brings the second arm 74 away from the first arm 64.

The alignment corrector 6 is configured to be placed on a head of a metatarsus, and the first arm 64 and second arm 74 are configured to allow a correction of a metatarsus varus.

All the components of the bone deformity repairing system of the present invention herein described may be made of radio-transparent material and constructed from biocompatible materials suitable for implantation, such as surgical-grade stainless steel, titanium alloys, or high-strength polymers. These materials may be chosen for their durability, resistance to corrosion, and compatibility with imaging techniques used in post-operative follow-up.

The components of the system may be designed with ergonomics in mind, featuring smooth edges and intuitive controls to facilitate ease of use in the operating room. The system may also be designed to be modular, allowing surgeons to select and use only the components necessary for each specific case.

The system may be accompanied by specialized instruments for installation and adjustment, which may be designed to minimize tissue damage and improve surgical outcomes. These instruments may include custom wrenches, guides, and measurement tools calibrated specifically for use with the system.

In practice, the system may be used in a staged approach. First, the dorsal gap reducer may be provided, with the first anchoring block 10 connected to the gap reducing block 20 through the longitudinal connecting bar 16 and the threaded rod 29, and the second anchoring block 30 loosely disposed on the longitudinal connecting bar 16. Then, the first anchoring block 10 may be anchored to a first bone through at least one surgical wire 13 and the second anchoring block 30 may be anchored to a second bone through the surgical pins 35. Then, second anchoring block 30 may be locked to the gap reducing block 20 and the gap reducing block 20 may be translated towards or away from the first anchoring block 10 in order to gradually reduce or increase the distance, the gap, between the bones. Then, second anchoring block 30 may be unlocked from the gap reducing block 20 and rotated around the longitudinal connecting bar 16 to create a pronation of the second bone. Then, second anchoring block 30 may be again locked to the gap reducing block 20.

Once the desired reduction is achieved, the positioning guide 4 may be used to place fixing elements, such as screws or pins, to maintain the corrected position. Finally, the alignment corrector 6 may be employed to make any necessary fine adjustments to ensure optimal alignment.

The bone deformity repairing system of the present invention may be configured to be compatible with various imaging modalities, including fluoroscopy and CT scanning, allowing for real-time visualization and adjustment during the procedure.

This feature may enhance the precision of the correction and may reduce the need for revision surgeries.

Post-operatively, the system of the present invention may be configured for some degree of micro-motion between the bones, which may promote healing and prevent stress shielding. The components may be configured to be either permanent implants or removable after healing is complete, depending on the specific needs of each patient.

The system may be configured to correct a wide range of bone deformities, including but not limited to hallux valgus, metatarsus adductus, and various midfoot deformities. Its versatility may make it a valuable tool in the arsenal of orthopedic surgeons specializing in foot and ankle reconstruction.

In summary, the system for repairing bone deformities, when comprising its three interconnected components, a dorsal gap reducer, a positioning guide for fixing elements, and an alignment corrector, may represent a comprehensive and adaptable solution for addressing complex bone deformities. These components may be configured to work in concert to address various aspects of bone deformity correction, providing a comprehensive solution for orthopedic surgeons and patients alike.

The design of the present system may allow for precise, controlled correction while potentially minimizing trauma to surrounding tissues, which may lead to improved patient outcomes and satisfaction.

## Claims

1. A bone deformity repairing system comprises a dorsal gap reducer (1) comprising a first anchoring block (10) configured to rotatably anchor the dorsal gap reducer (1) to a first bone, a gap reducing block (20) that is slidably connected to the first anchoring block (10) and a second anchoring block (30) that is rotatable with respect to and selectively lockable to the gap reducing block (20) and it is configured to be anchored to a second bone.

2. A bone deformity repairing system according to claim 1, **characterized in that** the first anchoring block (10) comprises a main body (11) and a connecting bar (16) longitudinally protruding from the main body (11), the gap reducing block (20) comprises a main body (21) having a hollow portion (23) provided with a seat (22), the connecting bar (16) of the first anchoring block (10) being inserted in the seat (22) of the hollow portion (23) to slidably connect the first anchoring block (10) to the gap reducing block (20).

3. A bone deformity repairing system according to claim 2, **characterized in that** the second anchoring block (30) is slidably and rotatably disposed on the longitudinal connecting bar (16) of the first anchoring block (10).

4. A bone deformity repairing system according to claim 3, **characterized in that** the second anchoring block (30) comprises an indentation (36) circumferentially distributed on an lateral side thereof, and an external surface of the hollow peripheral portion (23) of the gap reducing block (20) is provided with an indentation (26) circumferentially distributed and configured to match the indentation (36) of the second anchoring block (30).

5. A bone deformity repairing system according to any of the preceding claims, **characterized in that** the second anchoring block (30) comprises a radial slot (27) and the gap reducing block (20) comprises a prominence (24) disposed inside the slot (27).

6. A bone deformity repairing system according to any of the preceding claims, **characterized in that** the anchoring blocks (10, 30) comprise an anchoring group (12, 14, 32, 34) respectively.

7. A bone deformity repairing system according to claim 1, wherein the dorsal gap reducer (1) further comprises an actuating mechanism configurated to slide the gap reducing block (20) with respect to the first anchoring block (10) reducing or increasing the reciprocal distance therebetween.

8. A bone deformity repairing system according to claim 7, **characterized in that** the first anchoring block (10) comprises a seat (17) and the gap reducing block (20) comprises a threaded conduit (25), the actuating mechanism comprising a threaded rod (29) accommodated in the threaded conduit (25) and inserted in the seat (17).

9. A bone deformity repairing system according to any of the preceding claims, **characterized in that** it also comprises a positioning guide (4).

10. A bone deformity repairing system according to any of the preceding claims, **characterized in that** it also comprises an alignment corrector (6).
